# EUROPÄISCHE PATENTANMELDUNG

(11) **EP 4 520 248 A1**
(43) Veröffentlichungstag der Anmeldung: **12.03.2025**
(21) Anmeldenummer: 24197312.2
(22) Anmeldetag: 29.08.2024
(51) Int. Cl.: A61B 1/00, A61B 1/05, A61B 1/06, A61B 1/12, G02B 23/24

(54) **TRAEGERVORRICHTUNG ZUM VORMONTIEREN UND HALTEN EINER SENSORBAUGRUPPE UND ZUM EINSETZEN IN EINEN SCHAFT EINER MEDIZINISCHEN BILDGEBUNGSVORRICHTUNG, MEDIZINISCHE BILDGEBUNGSVORRICHTUNG UND VERFAHREN ZUM VORMONTIEREN**

(30) Priorität: 07.09.2023 DE 102023124089
(71) Anmelder: Karl Storz SE & Co. KG, 78532 Tuttlingen (DE)
(72) Erfinder: Eisenlauer, Johannes, 78532 Tuttlingen (DE)
(74) Vertreter: Weickert, Jonas

(57) **Zusammenfassung**

Die Erfindung betrifft eine Trägervorrichtung zum Vormontieren und Halten einer Sensorbaugruppe und zum Einsetzen in einen Schaft einer medizinischen Bildgebungsvorrichtung, wobei die Trägervorrichtung als eine jeweilige Seite eine distale Seite, eine proximale Seite, eine Oberseite, eine Unterseite und zwei sich gegenüberliegende Seiten aufweist, wobei die Sensorbaugruppe der Trägervorrichtung zuordenbar ist und mindestens einen Sensor aufweist und der Sensor an der distalen Seite der Trägervorrichtung montierbar ist, wobei die Trägervorrichtung von der proximalen Seite bis zur distalen Seite einen durchgehenden Hohlraum aufweist, sodass bei einer Vormontage des Sensors an der distalen Seite der Trägervorrichtung der Sensor mittels eines in den Hohlraum einführbaren Ausrichtwerkzeuges von innen in eine vorgegebene Position ausrichtbar ist. Des Weiteren betrifft die Erfindung eine medizinische Bildgebungsvorrichtung und ein Verfahren zum Vormontieren einer Sensorbaugruppe.

## Beschreibung

Die Erfindung betrifft eine Trägervorrichtung zum Vormontieren und Halten einer Sensorbaugruppe und zum Einsetzen in einen Schaft einer medizinischen Bildgebungsvorrichtung, wobei die Trägervorrichtung als eine jeweilige Seite eine distale Seite, eine proximale Seite, eine Oberseite, eine Unterseite und zwei sich gegenüberliegende Seiten aufweist, wobei die Sensorbaugruppe der Trägervorrichtung zuordenbar ist und mindestens einen Sensor aufweist und der Sensor an der distalen Seite der Trägervorrichtung montierbar ist. Des Weiteren betrifft die Erfindung eine medizinische Bildgebungsvorrichtung und ein Verfahren zum Vormontieren einer Sensorbaugruppe an eine Trägervorrichtung und zum Einsetzen in einen Schaft einer medizinischen Bildgebungsvorrichtung.

Medizinische Bildgebungsvorrichtungen, wie beispielsweise Exoskope oder Endoskope, werden zur Betrachtung einer Außenoberfläche und/oder eines Inneren eines menschlichen oder tierischen Körpers oder eines anderen zu untersuchenden, beispielsweise technischen, Objektes verwendet. Medizinische Bildgebungsvorrichtungen weisen üblicherweise mindestens einen Sensor und/oder Bildsensor auf. Im Falle von Endoskopen ist üblicherweise in einem distalen Endabschnitt eines Schaftes des Endoskops ein Objektivlinsensystem zur Erzeugung eines Bildes eines Betrachtungsbereiches angeordnet. Bei einem Videoendoskop ist häufig auch der elektronische Bildsensor zum Erzeugen des endoskopischen Bildes im distalen Endabschnitt innerhalb des Schaftes angeordnet, wobei dessen Sensorebene auf einer Bildebene des Objektivlinsensystems liegt. Derartige Videoendoskope werden als Chip-On-The-Tip-Endoskope (COTT-Endoskope) bezeichnet.

Aufgrund des geringen Durchmessers des Schaftes von üblicherweise wenigen Millimetern sind erhöhte Anforderungen an die Montage und die Befestigung des Objektivlinsensystems und des Sensors und/oder Bildsensors innerhalb des Hohlraum des Schaftes gestellt. Aufgrund der beengten Platzverhältnisse vor allem im distalen Endabschnitt des Schaftes besteht die Gefahr, dass das Objektivlinsensystem und/oder der Sensor und/oder Bildsensor nicht optimal zueinander ausgerichtet sind oder erst nach einer Endmontage bei der Werksprüfung des Endoskops festgestellt wird, dass der Sensor und/oder Bildsensor nicht funktionsfähig ist. Zudem muss der Sensor und/oder Bildsensor im Inneren des Schaftes befestigt, beispielsweise verklebt, werden. Nachteilig hierbei ist, dass undefinierte Kontaktstellen bei der Verklebung vorliegen können und der Klebstoff sich aufgrund der geringen Bauteildimensionen und der engen Einbauverhältnisse unerwünscht und unkontrolliert verteilen kann. Zudem besteht gerade bei der Fertigung von medizinischen Bildgebungsvorrichtungen für den einmaligen Gebrauch, wie beispielsweise ein Einweg-Endoskop, ein Bedarf an schnellen, einfachen und kostengünstigen Fertigungsverfahren. Dagegen muss beispielsweise bei einem wiederverwendbaren Endoskop das Montageverfahren derart sicher und verlässlich sein, dass der im distalen Endabschnitt montierte Sensor und/oder Bildsensor langfristig trotz sich regelmäßig wiederholender Reinigung und Entkeimung, beispielsweise durch Autoklavieren bei hohen Temperaturen und mit unter Druck stehendem Dampf, seine Position innerhalb des Schaftes beibehält und sich die Verklebung nicht löst.

Aufgabe der Erfindung ist es, den Stand der Technik zu verbessern.

Gelöst wird die Aufgabe durch eine Trägervorrichtung zum Vormontieren und Halten einer Sensorbaugruppe und zum Einsetzen in einen Schaft einer medizinischen Bildgebungsvorrichtung, wobei die Trägervorrichtung als eine jeweilige Seite eine distale Seite, eine proximale Seite, eine Oberseite, eine Unterseite und zwei sich gegenüberliegende Seiten aufweist, wobei die Sensorbaugruppe der Trägervorrichtung zuordenbar ist und mindestens einen Sensor aufweist und der Sensor an der distalen Seite der Trägervorrichtung montierbar ist, wobei die Trägervorrichtung von der proximalen Seite bis zur distalen Seite einen durchgehenden Hohlraum aufweist, sodass bei einer Vormontage des Sensor an der distalen Seite der Trägervorrichtung der Sensor mittels eines in den Hohlraum einführbaren Ausrichtungswerkzeuges von innen in eine vorgegebene Position ausrichtbar ist.

Somit wird eine Trägervorrichtung bereitgestellt, in, an und/oder auf der eine Sensorbaugruppe und/oder ein Sensor außerhalb der medizinischen Bildgebungsvorrichtung vormontierbar und/oder befestigbar ist. Folglich ist nach erfolgter Vormontage die Sensorbaugruppe und/oder der Sensor angeordnet an der Trägervorrichtung werkseitig auf Funktionsfähigkeit prüfbar, bevor die Trägervorrichtung mit der vormontierten Sensorbaugruppe und/oder dem Sensor in die medizinische Bildgebungsvorrichtung und insbesondere in einen distalen Endabschnitt eines Schaftes eingesetzt wird. Somit wird im Falle eines Videoendoskopes mit der Trägervorrichtung eine Vorbaugruppe bereitgestellt, welche außerhalb des Videoschaftes aufbaubar ist und auf ein funktionierendes Videobild prüfbar ist, bevor diese in den Videoschaft eingebaut wird.

Es ist besonders vorteilhaft, dass die Trägervorrichtung einen von ihrer proximalen Seite bis zu ihrer distalen Seite durchgehenden Hohlraum aufweist, welcher multifunktional während der Montage und dem späteren Betrieb der medizinischen Bildgebungsvorrichtung nutzbar ist. Dadurch kann bei der Vormontage der Sensor mittels eines in den Hohlraum eingeführten Ausrichtwerkzeuges von innen in eine vorgegebene Position und/oder räumliche Lage ausgerichtet werden. Prinzipiell ist es auch möglich, dass die Ausrichtung des Sensors nach Einsetzen der Trägervorrichtung mit der vormontierten Sensorbaugruppe und/oder dem Sensor innerhalb des distalen Endabschnittes nachjustiert wird, indem beispielsweise ein um 90° gebogenes Werkzeug verwendet und proximalseitig in den durchgehenden Hohlraum eingeführt wird. Somit ist nach Einsetzen der Trägervorrichtung mit der vormontierten Sensorbaugruppe in den Schaft noch eine Korrektur der Ausrichtung des vormontierten Sensors ermöglicht.

Es ist besonders vorteilhaft, dass sowohl die Trägervorrichtung selbst als auch ihr durchgehender Hohlraum nach der Vormontage der Sensorbaugruppe und dem Einsetzen in den Schaft andere Funktionen während des Betriebes der medizinischen Bildgebungsvorrichtung aufweisen können. Neben der Haltefunktion ist die Trägervorrichtung selbst auch als Wärmeübertragungselement nutzbar, um beispielsweise eine Betriebswärme des Sensors und/oder einer distalseitig angeordneten Lichtquelle abzuführen und/oder zu verteilen und eine Überhitzung des distalen Endabschnittes des Schaftes zu vermeiden.

Prinzipiell ist herauszustellen, dass der Sensor nicht zwingend an der distalen Seite der Trägervorrichtung montiert werden muss. Dadurch ist das Objektiv und/oder das Objektivlinsensystem auch aufteilbar und kann beispielsweise mit einigen Komponenten an der distalen Seite und mit anderen Komponenten an der proximalen Seite der Trägervorrichtung montiert werden, da der durchgehende Hohlraum eine optische Durchgängigkeit durch die Trägervorrichtung gewährleistet. Bei einer Aufteilung der Komponenten eines Objektivs oder eines Objektivlinsensystems auf die beiden distalen und proximalen Seiten der Trägervorrichtung ist der Sensor entsprechend an der proximalen Seite des aufgeteilten Objektivs und/oder Objektivlinsensystems anordenbar.

Ein wesentlicher Gedanke der Erfindung beruht darauf, mittels einer Trägervorrichtung nicht nur eine optimale Vormontage und/oder ein Halten der Sensorbaugruppe und/oder des Sensors bereits außerhalb der medizinischen Bildgebungsvorrichtung zu ermöglichen, sondern die Trägervorrichtung mittels des durchgehenden Hohlraumes auch derart auszubilden, dass diese sowohl bei der Montage als auch beim Betrieb der medizinischen Bildgebungsvorrichtung multifunktional nutzbar ist. Dadurch, dass mittels der Trägervorrichtung die Vormontage und die Funktionsprüfung außerhalb der medizinischen Bildgebungsvorrichtung ermöglicht sind, ist die Trägervorrichtung mit der vormontierten Sensorbaugruppe einfacher in die engen räumlichen Verhältnisse innerhalb des Schaftes einsetzbar und verlässlicher befestigbar. Folglich ist sowohl eine Wiederverwendung trotz einer wiederholten Reinigung und Sterilisation der Bildgebungsvorrichtung als auch eine einfache und kostengünstige Fertigung von Einweg-Endoskopen ermöglicht.

### Folgendes Begriffliche sei erläutert:

Eine "medizinische Bildgebungsvorrichtung" ist insbesondere jede Art von technischer und/oder elektrischer Vorrichtung, welche geeignet ist, ein Bild eines Betrachtungsbereiches in einem medizinischen Umfeld oder auch in einem technischen Umfeld aufzunehmen, zu verarbeiten und/oder weiterzuleiten. Bei einer medizinischen Bildgebungsvorrichtung kann es sich beispielsweise um ein Endoskop oder ein Exoskop handeln.

Ein "Exoskop" ist insbesondere ein medizinisches Gerät oder Instrument zum Beobachten und/oder Beleuchten eines Betrachtungsbereiches an einem Patienten von einer Stelle abseits des Körpers des Patienten. Bei einem Exoskop kann es sich auch um ein Auflichtmikroskop handeln. Ein "Endoskop" ist insbesondere ein medizinisches oder industrielles Gerät zur endoskopischen Untersuchung und Betrachtung einer menschlichen oder einer tierischen Körperhöhle und/oder eines industriellen Hohlraums, wie einem Rohr. Das Endoskop weist insbesondere ein Handstück, einen Schaft, eine Lichtquelle, einen Lichtleiter, einen Sensor und/oder eine Kamera auf. Bei dem Endoskop handelt es sich insbesondere um ein Videoendoskop, welches eine digitale Bildaufnahme und Bildübertragung aufweist. Bei einem Videoendoskop handelt es sich insbesondere ein Chip-on-the-Tip(COTT)-Endoskop, wobei der Bildsensor als ein Chip, beispielsweise ein CMOS-Chip, ausgebildet und am distalen Endabschnitt des Schaftes des Videoendoskopes angeordnet ist. Die durch den Bildsensor aufgenommenen Bilddaten sind insbesondere elektronisch durch den Schaft in proximaler Richtung zum Handstück und weiter an ein Anzeigesystem und/oder an eine Bildverarbeitungseinheit übertragbar, um das endoskopische Bild für den Benutzer darzustellen. Neben human- und tiermedizinischen Anwendungen kann ein Endoskop und/oder Videoendoskop auch zu industriellen Zwecken, beispielsweise zur Sichtprüfung in schwer zugänglichen Hohlräumen, eingesetzt werden. Bei industriellen Anwendungen wird ein Endoskop häufig als Boroskop bezeichnet.

Ein Endoskop oder Exoskop als Einrichtung an sich kann auch Bestandteil eines gesamten Endoskop- oder Exoskopsystems sein, welches weitere Einrichtungen, wie weitere Sensoren, Messgeräte, Kabel, eine Kamerakontrolleinheit, eine Lichtquelle, ein Anzeigegerät zum Anzeigen der erfassten Bilddaten, einen Monitor und ähnliches aufweisen kann.

Ein "Schaft" ist insbesondere eine langgestreckte und hohle Röhre zum Heranbringen an eine Körperoberfläche und/oder zum zumindest teilweisen Einbringen in eine Körperhöhle und/oder eine technische Öffnung. Bei einem Schaft kann es sich insbesondere um eine flexiblen und/oder starren Schaft handeln. Im Falle eines starren Schaftes ist insbesondere der distale Endabschnitt abwinkelbar ausgestaltet. Der Schaft weist insbesondere einen Durchmesser in einem Bereich von 2 mm bis 10 mm auf. Prinzipiell kann der Schaft jegliche Querschnittsform aufweisen. Bevorzugt weist der Schaft einen runden oder ovalen Querschnitt auf. In seinem Inneren kann der Schaft insbesondere weitere Komponenten, wie einen Lichtwellenleiter zur Beleuchtung eines Objektfeldes, einen Arbeitskanal oder mehrere Arbeitskanäle zum Zuführen von Spülflüssigkeit oder eines Werkzeuges, wie einer Biopsienadel oder eine Elektrode, aufweisen. Der Schaft ist an seinem proximalen Ende insbesondere mit einem Handstück der medizinischen Bildgebungsvorrichtung reversibel mechanisch und/oder elektrisch verbindbar oder festverbunden. In seinem distalen Endabschnitt weist der Schaft insbesondere eine verschließbare Öffnung auf, durch welche die Trägervorrichtung mit der vormontierten Sensorbaugruppe in den Hohlraum des Schaftes eingebracht und dort endmontiert werden kann. Die Öffnung im Endabschnitt des Schaftes ist insbesondere mittels eines Deckels, bevorzugt fluiddicht, verschließbar. Selbstverständlich kann die Öffnung im Schaft auch an jeder anderen Stelle im Schaft, beispielsweise in seiner Mitte oder seinem proximalen Endabschnitt, angeordnet sein.

Die "Längsrichtung" des Schaftes ist eine Richtung einer längsten Ausdehnung des Schaftes. Dementsprechend ist die "Längsachse" des Schaftes diejenige Achse, welche der Richtung seiner längsten Ausdehnung entspricht.

"Unter "distalseitig" und "distal" wird eine patientenkörpernahe und somit benutzerferne Anordnung und/oder ein entsprechendes Ende oder Abschnitt verstanden. Dementsprechend wird unter "proximalseitig" oder "proximal" eine benutzernahe und somit patientenkörperferne Anordnung und ein entsprechendes Ende oder Abschnitt verstanden. Somit ist die "proximale Richtung" eine Richtung gerichtet auf das proximale Ende der medizinischen Bildgebungsvorrichtung und/oder des Handstückes und/oder des Endoskopes. Dementsprechend ist die "distale Richtung" die entgegengesetzte Richtung gerichtet auf eine Spitze und/oder ein freies Ende des Schaftes.

Ein "Sensor" ist insbesondere ein technisches Bauteil, welches bestimmte physikalische oder chemische Eigenschaften und/oder die stoffliche Beschaffung seiner Umgebung qualitativ oder als Messgröße quantitativ erfasst. Bei einem Sensor kann es sich prinzipiell um jegliche Art von Sensor handeln, wie einen mechanischen, thermoelektrischen, resistiven, piezoelektrischen, kapazitiven, induktiven, optischen, akustischen oder magnetischen Sensor. Somit kann es sich beispielsweise bei dem Sensor um einen Beschleunigungssensor oder einen Drucksensor handeln. Bevorzugt handelt es sich bei dem Sensor um einen optischen Sensor und/oder einen Bildsensor.

Ein "Bildsensor" ist insbesondere ein lichtempfindliches elektronisches Bauelement, welches auf einem inneren Photoeffekt beruht. Mittels des Bildsensors wird insbesondere ein Bild oder werden mehrere Bilder aus dem Betrachtungsbereich der medizinischen Bildgebungsvorrichtung aufgezeichnet und in elektronische Signale umgewandelt. Der Bildsensor weist eine Sensorebene in der Bildebene des optischen Systems, des Linsensystems und/oder des Objektivs der medizinischen Bildgebungsvorrichtung auf. Bei einem elektronischen Bildsensor kann es sich insbesondere um einen CCD-Sensor (charge-coupled device) oder um einen CMOS-Sensor (complementary metal oxide-semiconductor) handeln. Bevorzugt ist der elektronische Bildsensor direkt als Chip im distalen Endabschnitt und/oder der Spitze des Schaftes der medizinischen Bildgebungsvorrichtung und/oder des Videoendoskopes angeordnet und überträgt die digitalen Bildsignale vom distalen Ende des Schaftes zum proximalen Ende mittels elektrische Übertragungsleitungen.

Eine "Lichtquelle" ist insbesondere ein Bauteil oder ein Gerät, von dem Licht emittiert wird. Die Lichtquelle kann als eigenständiges Gerät ausgebildet sein, wobei Licht über einen Verbindungsanschluss und einen Lichtleiter in den Schaft und bis zu seinem distalen Ende eingekoppelt wird. Ebenso kann es sich bei der Lichtquelle insbesondere um eine LED-Lichtquelle und/oder eine Kaltlichtquelle handeln. Die Lichtquelle kann direkt in der medizinischen Bildgebungsvorrichtung, beispielsweise im Handstück oder im Schaft, integriert sein. Bevorzugt ist die Lichtquelle als LED oder als eine andere lichterzeugende Einrichtung direkt im distalen Endabschnitt des Schaftes und/oder der medizinischen Bildgebungsvorrichtung ausgebildet. Das Licht der Lichtquelle dient insbesondere zum homogenen Ausleuchten des Untersuchungsgebietes und des gewünschten Betrachtungsbereiches.

Ein "Betrachtungsbereich" ist insbesondere ein Bereich, ein Volumen, eine Außenoberfläche und/oder eine Innenoberfläche eines menschlichen oder tierischen Körpers oder eines technischen Objektes. Aus dem gesamten Betrachtungsbereich oder einem Teil des Betrachtungsbereiches wird insbesondere mittels eines Objektivs eine optische Abbildung erzeugt und/oder mittels des Bildsensors erfasst. Bei einem Betrachtungsbereich kann es sich beispielsweise um eine äußere Hautoberfläche, ein inneres Organ, einen natürlichen Hohlraum in einem menschlichen oder tierischen Körper oder um das Innere einer technischen Rohrleitung handeln.

Unter einer "Trägervorrichtung" wird insbesondere eine Vorrichtung zum Vormontieren, Tragen und/oder Halten einer Sensorbaugruppe verstanden. Bei der Trägervorrichtung kann es sich insbesondere um ein Bauteil oder eine Bauteilgruppe handeln. Die Trägervorrichtung kann insbesondere einteilig oder mehrteilig ausgebildet sein. Prinzipiell kann die Trägervorrichtung jegliche Form aufweisen. Die Trägervorrichtung weist jedoch insbesondere Außenmaße auf, welche geringer sind als die Abmessungen des Hohlraums innerhalb des Schaftes, in welchem die Trägervorrichtung und/oder die vormontierte Sensorbaugruppe aufgenommen werden soll oder sollen. Die Trägervorrichtung ist insbesondere in Längsrichtung des Schaftes mit einer größeren Länge als ihrem Querschnitt ausgebildet. Die Trägervorrichtung weist insbesondere eine Länge in einem Bereich von 1,0 mm bis 8,0 mm, bevorzugt von 2,0 mm bis 4,0 mm, auf. Die Trägervorrichtung weist insbesondere eine Breite in einem Bereich von 1,0 mm bis 4,0 mm, bevorzugt von 2,5 mm bis 3,5 mm, auf. Die Trägervorrichtung weist insbesondere eine Höhe in einem Bereich von 1,0 mm bis 4,0 mm, bevorzugt in einem Bereich von 1,5 mm bis 2,5 mm, auf.

Unter einer "Seite" der Trägervorrichtung wird insbesondere sowohl eine Fläche als auch eine Richtungsangabe verstanden. Da die Trägervorrichtung prinzipiell jegliche äußere Form, wie beispielsweise ganz oder teilweise rechteckig, rund, zylindrisch und ähnliches, aufweisen kann, muss es sich bei einer Seite nicht um eine eindeutig abgegrenzte Seite mit Kanten handeln. Die jeweils aneinanderliegenden Seiten an der Außenoberfläche der Trägervorrichtung können auch ineinander übergehen, beispielsweise abgerundet sein. Somit wird unter einer Seite auch eine Fläche oder ein Flächenabschnitt der Trägervorrichtung verstanden, welche in eine bestimmte Richtung ausgerichtet ist. Somit ist die Oberseite insbesondere nach oben, die Unterseite nach unten, die zwei sich gegenüberliegenden Seiten rechts und links angeordnet und die distale Seite nach vorne oder nach erfolgtem Einbau in den Schaft ausgerichtet zum distalen Ende des Schaftes und entsprechend die proximale Seite nach hinten oder im eingebauten Zustand zum proximalen Ende des Schaftes ausgerichtet. Die jeweilige Seite der Trägervorrichtung kann eben, schräg oder sich konisch in eine Richtung erweiternd oder verengend ausgebildet sein. Die distale Seite der Trägervorrichtung, an welcher bevorzugt der Sensor und/oder Bildsensor montiert oder angeordnet ist oder wird, kann insbesondere schräg ausgebildet sein. Bevorzugt verläuft die distale Seite von der Unterseite schräg nach oben in proximaler Richtung zur Oberseite. Die Trägervorrichtung kann prinzipiell sämtliche Arten von Material aufweisen. Beispielsweise weist die Trägervorrichtung Kunststoff und/oder Metall auf. Neben Edelstahl kann die Trägervorrichtung insbesondere Kupfer und/oder Messing aufweisen, um eine Wärmeableitung durch die Trägervorrichtung selbst zu ermöglichen. Die Trägervorrichtung weist mindestens einen von ihrer proximalen Seite zur distalen Seite durchgehenden Hohlraum auf. Die Trägervorrichtung kann aber auch zwei oder mehrere durchgehende Hohlräume und/oder Kanäle aufweisen. Ergänzend und/oder anstelle des mindestens einen durchgehenden Hohlraums kann die Trägerborrichtung jedoch auch eine in Längsrichtung durchgehende Aussparung an ihrer Außenoberfläche aufweisen.

Ein "Hohlraum" ist insbesondere ein leerer und/oder ausgesparter Raum, zumindest vor der Vormontage, im Inneren der Trägervorrichtung. Nach der Vormontage der Trägervorrichtung kann in den durchgehenden Hohlraum der Trägervorrichtung insbesondere ein Ausrichtwerkzeug und/oder ein anderes Bauteil vollständig oder partiell angeordnet sein. In dem Hohlraum der Trägervorrichtung kann insbesondere auch mindestens eine Wärmeübertragungselement angeordnet sein. Der Hohlraum ist insbesondere konzentrisch zur Längsmittelachse der Trägervorrichtung und/oder des Schaftes angeordnet. Der durchgehende Hohlraum oder mehrere durchgehende Hohlräume können jedoch auch exzentrisch zur Längsmittelachse der Trägervorrichtung und/oder des Schaftes angeordnet sein. Der Hohlraum kann insbesondere sämtliche Formen aufweisen, beispielsweise stabförmig mit einem rechteckigen oder runden Querschnitt. Bei dem Hohlraum kann es sich beispielsweise um eine durchgehende Bohrung handeln. Der Hohlraum weist insbesondere einen Durchmesser in einem Bereich von 0,1 mm bis 3,0 mm, bevorzugt von 0,5 mm bis 2,0 mm, auf.

Eine "Sensorbaugruppe" ist insbesondere ein aus zwei oder mehreren Teilen oder Baugruppen niederer Ordnung bestehender Gegenstand. Die Sensorbaugruppe weist zumindest einen Sensor auf. Die Sensorbaugruppe ist insbesondere montierbar und/oder demontierbar. Die Sensorbaugruppe ist mit ihren einzelnen Bestandteilen insbesondere an, auf und/oder in der Trägervorrichtung montierbar und/oder anordenbar, sodass im vormontierten Zustand die Sensorbaugruppe und die Trägervorrichtung als eine gemeinsame Baugruppe vorliegen (auch aufgebaute Vorbaugruppe genannt).

Unter einem "Ausrichtwerkzeug" wird jegliche Art von Werkzeug verstanden, mit dem eine Position und/oder eine Orientierung des Sensors an der Trägervorrichtung einstellbar ist. Bei einem Ausrichtwerkzeug kann es sich beispielsweise um einen Dorn oder einen Haken handeln. Das Ausrichtwerkzeug wird insbesondere durch die proximalseitige Öffnung des durchgehenden Hohlraums in den Hohlraum eingeschoben und wirkt mit seinem distalen Ende auf die Rückseite des Sensors und/oder des Sensorhalters ein. Dazu kann der Sensor und/oder der Sensorhalter auf seiner Rückseite und somit auf seiner in proximaler Richtung ausgerichteten Seite entsprechende Gegenaufnahmen und/oder Aussparungen aufweisen, in welche die Spitze des Ausrichtwerkzeuges und/oder Dornes eingreifen kann.

In einer Ausführungsform der Trägervorrichtung weist oder weisen die Sensorbaugruppe und/oder die Trägervorrichtung den Sensor, eine Objektivfassung, ein Objektiv und/oder einen Träger für elektronische Bauteile auf.

Somit kann, je nach Art des Sensors und der benötigten Bauteile, die Sensorbaugruppe zunächst alleine und/oder direkt an der Trägervorrichtung vormontiert werden.

Ein "Objektiv" ist insbesondere ein sammelndes optisches System, welches eine reelle optische Abbildung eines Gegenstandes und/oder Betrachtungsfeldes erzeugt. Das Objektiv weist mindestens eine Linse auf. Des Weiteren kann das Objektiv auch zwei oder mehrere Linsen und eine Blende aufweisen. Distalseitig kann das Objektiv hinter einem Deckglas angeordnet sein, wobei das Deckglas am distalen Ende den Schaft angrenzt, oder das Deckglas kann auch Bestandteil des Objektives sein. Bei dem Objektiv handelt es sich insbesondere um ein ultrakurzes Objektiv. Bevorzugt ist das Objektiv direkt hinter dem Deckglas angeordnet und mit seiner proximalen Seite an der distalen Seite eines Bildsensors angebracht. Das Objektiv kann auch zwei oder mehrteilig und/oder örtlich verteilt, beispielsweise auf der distalen Seite und der proximalen Seite der Trägervorrichtung, angeordnet sein.

Die "Objektivfassung" ist insbesondere ein Gehäuse und/oder ein Halter für das Objektiv. Die Objektivfassung umgibt insbesondere teilweise oder vollständig die optischen Bestandteile des Objektives. Unter der Objektivfassung wird auch die Stelle verstanden, an der das Objektiv mit einem weiteren Bestandteil, wie beispielsweise dem Deckglas oder dem Sensor, verbunden ist.

Bei einem "Träger für elektronische Bauteile" kann es sich insbesondere um eine Leiterplatte, eine Platine und/oder eine gedruckte Schaltung handeln. Der Träger für die elektronischen Bauteile weist insbesondere elektrische Anschlüsse für den Sensor und/oder weitere Bauteile der Sensorbaugruppe auf, welche mittels Spannung versorgt werden sollen. Der Träger dient insbesondere sowohl zur mechanischen Befestigung als auch zur elektrischen Verbindung der Bestandteile der Sensorbaugruppe im montierten Zustand. Der Träger für die elektronischen Bauteile wird auf eine Seite oder den Abschnitt einer Seite, insbesondere auf die Oberseite oder die Unterseite, der Trägervorrichtung aufgeklebt.

Um die beengten Platzverhältnisse im Hohlraum des distalen Endabschnittes des Schaftes optimal auszunutzen und eine elektrische Verbindung zwischen der proximalen Seite der Trägervorrichtung und dem Handstück der Bildgebungsvorrichtung sowie eine Spannungsversorgung des angeordneten Sensors zu ermöglichen, ist der Träger für elektronische Bauteile an der Oberseite oder der Unterseite der Trägervorrichtung angeordnet.

Zudem liegt durch diese Anordnung an der Ober- oder Unterseite der Trägervorrichtung der Träger für elektronische Bauteile nicht im optischen Strahlengang, sodass das Objektiv auch beidseitig getrennt an der distalen Seite und der proximalen Seite der Trägervorrichtung angeordnet werden kann und dementsprechend ein Bildsensor dann proximalseitig des proximalen Teils des Objektivs angeordnet ist.

In einer weiteren Ausführungsform kann die Trägervorrichtung mindestens ein Wärmeübertragungselement aufweisen oder ist als ein Wärmeübertragungselement ausgebildet.

Um eine optimale Wärmeabführung und/oder -verteilung zu realisieren, kann das mindestens eine Wärmeübertragungselement zumindest teilweise in dem Hohlraum angeordnet sein, sodass eine Betriebswärme des Sensors in einer proximalen Richtung abführbar ist.

Bei dem "Wärmeübertragungselement" handelt es sich insbesondere um ein wärmeleitfähiges Element und/oder Bauteil und/oder einen wärmeleitfähigen Stoff. Das Wärmeübertragungselement und/oder die Trägervorrichtung selbst weist insbesondere ein wärmeleitendes Material, wie beispielsweise Metall, auf. Bei dem Wärmeübertragungselement kann es sich beispielsweise auch um einen Kühlkörper oder ein Kühlelement handeln. Beispielsweise ist eine Kupferscheibe als Kühlelement proximalseitig der proximalen Öffnung des durchgehenden Hohlraums angeordnet und der durchgehende Hohlraum ist mit Luft befüllt. Auch kann das Wärmeübertragungselement als Kupferdraht oder - stab ausgebildet sein, welcher in dem Hohlraum in Längsrichtung angeordnet ist und distalseitig die Rückseite des Sensors und/oder des Sensorhalters kontaktiert. Bei dem Wärmeübertragungselement kann es sich beispielsweise auch um ein Kupferrohr handeln, welches in dem Hohlraum der Trägervorrichtung angeordnet ist und selbst mit Luft gefüllt ist. Ebenso kann der Hohlraum der Trägervorrichtung nach der Montage mit einer Wärmeleitpaste gefüllt sein. Das Wärmeübertragungselement ist insbesondere über eine seiner Kontaktflächen direkt mit dem wärmeabgebenden Bauteil, wie beispielsweise dem Sensor, einer Lichtquelle und/oder einem anderen elektronischen Bauteil, gekoppelt. Ebenso kann die Wärmeübertragung und/oder -kopplung indirekt über ein weiteres Bauteil, den mit Luft gefüllten Hohlraum der Trägervorrichtung und/oder eine Wärmeleitpaste erfolgen. Das Wärmeübertragungselement überträgt die aufgenommene Wärme insbesondere weiter in proximaler Richtung an einen Wärmeleitkörper als Wärmesenke. Bei einem Wärmeleitkörper kann es sich beispielsweise um ein Wärmerohr (auch Heatpipe genannt) handeln. Das Wärmerohr ist insbesondere in proximaler Richtung entlang der Längsachse des Schaftes angeordnet und geführt. Das Wärmeübertragungselement kann insbesondere wieder direkt oder indirekt mit dem Wärmeleitkörper wärmetechnisch gekoppelt sein.

Unter "Betriebswärme" wird insbesondere die Wärme oder Wärmemenge verstanden, welche aufgrund des Betriebes des Sensors, Bildsensors, einer Lichtquelle und/oder eines anderen Bauteils aufgrund einer Temperaturerhöhung entsteht. Die Betriebswärme wird beispielsweise durch eine Verlustleistung des Sensors und/oder der Lichtquelle erzeugt und aufgrund eines Temperaturgradienten zur Umgebung abgegeben.

In einer weiteren Ausführungsform kann die Trägervorrichtung in mindestens einer Seite der jeweiligen Seiten mindestens eine Aussparung zum Aufnehmen eines Klebemittels aufweisen, sodass die Sensorbaugruppe an die Trägervorrichtung außerhalb des Schaftes vormontierbar und anschließend in den Hohlraum des Schaftes einsetzbar und verklebbar ist.

Somit kann die Trägervorrichtung mit der vormontierten Sensorgruppe als eine Einheit in den Schaft eingesetzt und anschließend an einer Innenwand des Schaftes mittels der mindestens einen Aussparung und eines Klebemittels verklebt und dadurch räumlich fixiert werden.

Um eine definierte Befestigung der Trägervorrichtung mit der vormontierten Sensorbaugruppe und eine definierte Menge des Klebemittels bereitzustellen, kann die Trägervorrichtung zwei Aussparungen, drei Aussparungen, vier Aussparungen und optional weitere Aussparungen aufweisen.

Durch die Positionen und die jeweilige Ausbildung der Aussparungen werden sowohl der jeweilige Ort der Verklebung als auch die Menge des Klebemittels vorgegeben und dadurch der Prozess vereinfacht. Zudem können ein Verschmieren und ein unerwünschter Austrag des Klebemittels in andere Bereiche des Hohlraums des Schaftes vermieden werden. Folglich wird sowohl die Anbindung der Trägervorrichtung innerhalb des Schaftes als auch die Dosierung des Klebemittels verbessert. Durch die Aussparung oder zwei oder mehrere Aussparungen wird ein definiertes Reservoir des Klebemittels bereitgestellt, welches ein Verkleben ohne Verschmieren innerhalb des Schaftes gewährleistet. Bevorzugt wird das Klebemittel bei noch offenem Schaft in die jeweilige Aussparung eingebracht, beispielsweise mittels einer Kanüle, nachdem die Trägervorrichtung mit der vormontierten Sensorbaugruppe in die vorgesehene Position innerhalb des Hohlraums des Schaftes eingesetzt worden ist. Alternativ zu einem Verkleben kann die Trägervorrichtung mittels der Aussparung oder der Aussparungen jedoch auch in dem Inneren des Schafts eingegossen sein und/oder werden.

In einer weiteren Ausführungsform der Trägervorrichtung sind die Aussparungen an den sich gegenüberliegenden Seiten angeordnet.

Somit sind de Aussparungen bei der eingesetzten Trägervorrichtung in dem Schaft in der distalen Richtung ausgerichtet seitlich links und rechts angeordnet und die Trägervorrichtung seitlich verklebt.

Um die Verbindung zwischen der Trägervorrichtung und einer Innenoberfläche des Schaftes weiter zu verbessern und/oder eine Kraftübertragung oder -aufnahme zu ermöglichen, kann zwischen zwei Aussparungen eine Rippe angeordnet sein, wobei mittels einer Außenoberfläche der Rippe eine Innenoberfläche des Schaftes kontaktierbar ist.

Dadurch wird über eine Rippe oder werden über mehrere Rippen eine jeweils definierte Anliegefläche der Trägervorrichtung an die Innenoberfläche des Schaftes und/oder eine definierte Verklebefläche bereitgestellt.

Eine "Aussparung" ist insbesondere ein ausgesparter Raum an und/oder in der Außenoberfläche der Trägervorrichtung. Bei einer Aussparung handelt es sich insbesondere um einen Einschnitt und somit eine Vertiefung in der Außenoberfläche der Trägervorrichtung. Prinzipiell kann die Aussparung und/oder können die Aussparungen an jeder Seite der Trägervorrichtung und/oder an jedem Abschnitt ihrer Außenoberfläche angeordnet sein. Bevorzugt ist die Aussparung oder sind die Aussparungen an den beiden gegenüberliegenden Seiten und somit an den linken und rechten Seiten der Trägervorrichtung bei Ausrichtung in distaler Richtung angeordnet. Die Aussparung oder die Aussparungen sind insbesondere in einer Längsrichtung von der Oberseite zur Unterseite ausgebildet. Prinzipiell muss die Aussparung in ihrer Längsrichtung nicht durchgehend von der Oberseite bis zur Unterseite ausgebildet sein. Die Aussparung kann beispielsweise auch an der Oberseite und/oder der Unterseite geschlossen und nur seitlich zur Innenoberfläche des Schaftes geöffnet sein. Auch kann die geschlossene Oberseite oder Unterseite eine kleine Durchgangsöffnung aufweisen, um gezielt das Klebemittel beispielsweise mit einer Kanüle durch diese kleine Durchgangsöffnung in die Aussparung einzubringen. Die Aussparung kann prinzipiell sämtliche Formen aufweisen. Ebenso können zwei oder mehrere Aussparungen gleiche Formen, Abmessungen und/oder Ausrichtungen oder entsprechend unterschiedliche Ausrichtungen, Formen und/oder Abmessungen aufweisen. Die Aussparung und/oder die Aussparungen kann oder können insbesondere im Querschnitt sämtliche Formen aufweisen, wie beispielsweise rechteckig oder halbkreisförmig. Die jeweilige Aussparung kann beispielsweise durch Fräsen in die Außenoberfläche der Trägervorrichtung gefertigt sein. Zwischen zwei Aussparungen kann insbesondere eine Rippe der Trägervorrichtung stehenbleiben oder speziell ausgebildet sein. Die Rippe weist insbesondere eine definierte Außenoberfläche und/oder Kontaktfläche auf, welche eine Innenoberfläche des Schaftes kontaktieren kann und/oder mit dieser verklebt ist. Sowohl die Oberfläche der jeweiligen Aussparung als auch die Außenoberfläche der Rippe kann oder können glatt, rau und/oder strukturiert ausgebildet sein. Diese Oberflächen können auch sowohl teilweise glatt, rau und/oder strukturiert ausgebildet sein. Ebenso können an der Oberfläche der Aussparung, beispielsweise zu ihrem Rändern hin, Strukturelemente angeordnet sein, welche zunehmend in die Aussparung hineinreichen und somit ein Wegfließen des Klebemittels verhindern. Durch eine solche Strukturierung der Außenoberfläche der Aussparung und/oder mittels einer jeweiligen Rippe können jeweils separate Klebetaschen bereitgestellt werden, zwischen denen ein Verschmieren und/oder ein Übergang des Klebemittels verhindert ist.

Bei einem "Klebemittel" (auch Klebstoff genannt) handelt es sich insbesondere um einen nicht metallischen Stoff, mit welchem die Werkstoffe der Trägervorrichtung und des Schaftes durch Oberflächenhaftung und mittels seiner inneren Festigkeit verbunden werden. Prinzipiell ist jegliches Klebemittel geeignet, welches eine Verbindung der jeweiligen Werkstoffe der Trägervorrichtung und des Schaftes ermöglicht. Durch das Klebemittel wird insbesondere eine stoffschlüssige Verbindung zwischen der Trägervorrichtung und der Innenoberfläche des Schaftes realisiert. Um unerwünschte Reflexionen und somit Störungen in dem erfassten Bild zu verhindern, kann das Klebemittel schwarz angefärbt sein. Bei dem Klebemittel kann es sich beispielsweise um einen lichthärtenden Klebstoff (auch UV-Kleber genannt) handeln, welcher ein schnelles Aushärten der Verbindung ermöglicht.

In einem weiteren Aspekt der Erfindung wird die Aufgabe gelöst durch eine medizinische Bildgebungsvorrichtung, insbesondere Videoendoskop mit einem Handstück und einem langgestreckten Schaft, wobei die medizinische Bildgebungsvorrichtung eine Lichtquelle zum Beleuchten eines Betrachtungsbereiches und der langgestreckte Schaft einen Hohlraum, einen elektrischen Leiter und/oder einen Arbeitskanal aufweist, wobei die medizinische Bildgebungsvorrichtung eine zuvor beschriebene Trägervorrichtung aufweist und der Sensor als ein elektronischer Bildsensor zum Aufnehmen des Betrachtungsbereiches ausgebildet ist.

Somit wird eine medizinische Bildgebungsvorrichtung und vor allem ein Videoendoskop bereitgestellt, bei der oder dem die Sensorgruppe an der Trägervorrichtung außerhalb des Schaftes vormontierbar ist und im vormontierten Zustand außerhalb des Gerätes selbst die Funktionsprüfung des Bildsensors durchgeführt werden kann. Dadurch sind Funktionsstörungen des montierten Bildsensors frühzeitig erkennbar, bevor dieser innerhalb des engen Schaftes endmontiert ist. Somit wird die Montage vereinfacht und ist kostengünstiger durchführbar. Zudem wird ein nicht funktionsfähiger und/oder nicht der Spezifikation entsprechend montierter Bildsensor erkannt, kann noch außerhalb des Videoendoskopes ausgetauscht werden und folglich muss nicht nachträglich das gesamte Videoendoskop aufgrund einer nachträglich festgestellten, nicht vorhandenen Funktionsfähigkeit oder falschen Ausrichtung des Bildsensors entsorgt werden.

Um die Trägervorrichtung mit der vormontierten Sensorbaugruppe als ein Bauteil in einfacher Weise in den Schaft einsetzen zu können, kann der Schaft eine verschließbare Öffnung zum Einsetzen der Trägervorrichtung und/oder der Sensorbaugruppe aufweisen.

Die Öffnung weist insbesondere zumindest eine etwas größere Abmessungen als die Außenabmessungen der Trägervorrichtung mit der vormontierten Sensorbaugruppe auf. Die Öffnung ist insbesondere mittels eines Deckels verschließbar. Hierbei kann der Deckel beispielsweise ebenfalls mittels eines Klebemittels oder mittels des für die Aussparung verwendeten Klebemittels an einer Kontaktfläche um die Öffnung verklebt sein oder werden. Ebenso kann der Deckel verschweißt sein.

In einer weiteren Ausführungsform der medizinischen Bildgebungsvorrichtung ist in dem Schaft mindestens ein Wärmeleitrohr ausgerichtet in distaler Richtung derart angeordnet, dass das mindestens eine Wärmeübertragungselement der Trägervorrichtung wärmeübertragend mit dem Wärmeleitkörper gekoppelt ist.

Somit ist mittels des Wärmeleitkörpers gezielt die aufgenommene Wärme des Wärmeübertragungselementes, insbesondere in proximaler Richtung, abführbar und/oder ableitbar. Hierbei kann eine direkte Wärmekopplung zwischen dem Wärmeübertragungselement und dem Wärmeleitkörper bestehen, sodass diese eine gemeinsame Kontaktfläche aufweisen. Ebenso kann eine indirekte Wärmekopplung vorliegen, indem beispielsweise zwischen dem proximalen Ende des Wärmeübertragungselementes und dem distalen Ende des Wärmeleitkörpers eine Wärmeleitpaste angeordnet ist.

In einem weiteren Aspekt der Erfindung wird die Aufgabe gelöst durch ein Verfahren zum Vormontieren einer Sensorbaugruppe an einer Trägervorrichtung und zum Einsetzen in einen Schaft einer medizinischen Bildgebungsvorrichtung, wobei die Sensorbaugruppe mindestens einen Sensor aufweist, die Trägervorrichtung als eine jeweilige Seite eine distale Seite, eine proximale Seite, eine Oberseite, eine Unterseite und zwei sich gegenüberliegende Seiten aufweist und die Trägervorrichtung von der proximalen Seite bis zur distalen Seite einen durchgehenden Hohlraum aufweist, mit folgenden Schritten:
- Verbinden eines Objektivs an dem Sensor, sodass das Objektiv distalseitig von dem Sensor angeordnet ist,
- Verbinden des Sensors mit einer distalen Seite der Trägervorrichtung, und
- Optional Einführen eines Ausrichtwerkzeuges in den Hohlraum der Trägervorrichtung in einer distalen Richtung und Ausrichten des Sensors von innen an der distalen Seite der Trägervorrichtung mittels eines Ausrichtwerkzeuges, und
- Einsetzen der Trägervorrichtung in den Schaft der medizinischen Bildgebungsvorrichtung.

Folglich wird ein Verfahren bereitgestellt, mit welchem in einfacher und kostengünstiger Weise eine Sensorbaugruppe mit mindestens einem Sensor in, auf und/oder an der Trägervorrichtung vormontiert und anschließend diese als vormontierte Vorbaugruppe in den Schaft der medizinischen Bildgebungsvorrichtung eingesetzt werden.

Das Verfahren wird insbesondere mit einer zuvor beschriebenen Trägervorrichtung und/oder einer zuvor beschriebenen medizinischen Bildgebungsvorrichtung durchgeführt.

In einer weiteren Ausgestaltungsform des Verfahrens wird oder werden vor dem Einsetzen in den Schaft ein Verbinden des Trägers für elektronischer Bauteile mit einer Oberseite oder Unterseite der Trägervorrichtung und/oder ein Einführen eines Wärmeübertragungselementes zumindest teilweise in den Hohlraum der Trägervorrichtung durchgeführt.

Um die vormontierte Vorbaugruppe und/oder die Trägervorrichtung mit der Sensorgruppe definiert in den Hohlraum des Schaftes anzuordnen und zu befestigen, wird vor oder nach dem Einsetzen ein Einbringen eines Klebemittels in mindestens eine Aussparung in einer Seite der Trägervorrichtung durchgeführt, sodass die Trägervorrichtung mit einer Innenseite des Schaftes verklebt wird.

Im Weiteren wird die Erfindung anhand von Ausführungsbeispielen näher erläutert. Es zeigen
- Figur 1: eine schematische partielle dreidimensionale Ansicht eines Videoendoskops und eines Anzeigesystems,
- Figur 2: eine dreidimensionale Darstellung eines Sensorhalterung in Draufsicht auf ihre distale Seite,
- Figur 3: eine schematische Seitenansicht einer montierten Sensorbaugruppe an der Sensorhalterung aus Figur 2,
- Figur 4: eine schematische dreidimensionale Darstellung der auf der Sensorhalterung vormontierten Sensorbaugruppe eingesetzt in einen distalen Endabschnitt eines Schaftes in Sicht von oben durch eine Montageöffnung,
- Figur 5: eine schematische dreidimensionale Seitenansicht der vormontierten und eingesetzten Sensorbaugruppe an der Sensorhalterung aus Figur 4 mit einem proximalseitig angeordneten Wärmeleitrohr, und
- Figur 6: eine Fließbilddarstellung von Schritten eines Verfahrens zum Vormontieren einer Sensorbaugruppe.

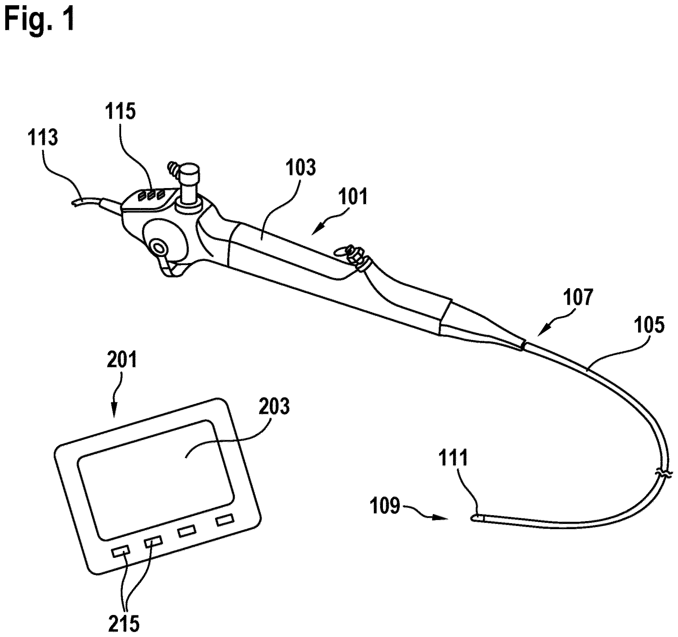

Ein Videoendoskop 101 umfasst ein Handstück 103 und einen länglichen Schaft 105, die an einem proximalen Ende 107 des Schafts 105 miteinander verbunden sind. Der Griff 103 umfasst Bedienelemente 115 und ist über ein Kabel 113 an seinem proximalen Ende mit einer externen, nicht gezeigten Steuer- und Verarbeitungseinheit und mit einem Anzeigesystem 201, das in Figur 1 gezeigt ist, verbunden. Das Anzeigesystem 201 weist einen Monitor 203 zum Anzeigen von endoskopischen Bildern und Bedienelemente 215.

Das Videoendoskop 101 ist dazu ausgebildet, Video- und Bilddaten von einem Objektivfeld innerhalb eines Hohlraums eines nicht gezeigten Körpers bereitzustellen. Dafür umfasst der längliche Schaft 105 an seinem distalen Ende 109 einen distalen Endabschnitt 111.

Eine Sensorhalterung 300 weist eine Oberseite 301 und eine gegenüberliegende Unterseite 303 auf. In einer distalen Richtung 317 betrachtet weist die Sensorhalterung 300 eine proximale Seite 307 und eine gegenüberliegende distale Seite 305 sowie eine rechte Seite 309 und eine linke Seite 311 auf. Von der proximalen Seite 307 zur distalen Seite 305 ist innenliegend ein durchgehender Hohlraum 313 in der Sensorhalterung 300 angeordnet. Der Hohlraum 313 ist mit seinem runden Querschnitt konzentrisch zu einer Längsmittelachse der Sensorhalterung 300 angeordnet. Der Querschnitt des Hohlraums 313 weist einen Durchmesser von 1,6 mm auf. Eine Breite der distalen Seite 305 beträgt 2,85 mm. Eine Länge der Sensorhalterung 300 entlang der Oberseite 303 beträgt 3,0 mm. Eine Höhe der Sensorhalterung 300 beträgt 2,0 mm. Die distale Seite 305 ist schräg zur Querebene senkrecht stehend auf der Längsachse des Sensorhalters 300 angeordnet, sodass die Oberseite 301 der schrägen distalen Seite 305 in proximaler Richtung (entgegen der distalen Richtung 317) zurückversetzt ist.

Auf der rechten Seite 309 und der linken Seite 311 weist die Sensorhalterung 300 jeweils drei Rippen 319 auf, wobei zwischen den Rippen 319 auf der linken Seite 311 eine erste Aussparung 321 und eine zweite Aussparung 322 und auf der rechten Seite 309 eine dritte Aussparung 323 und eine vierte Aussparung 324 angeordnet sind (Figur 1).

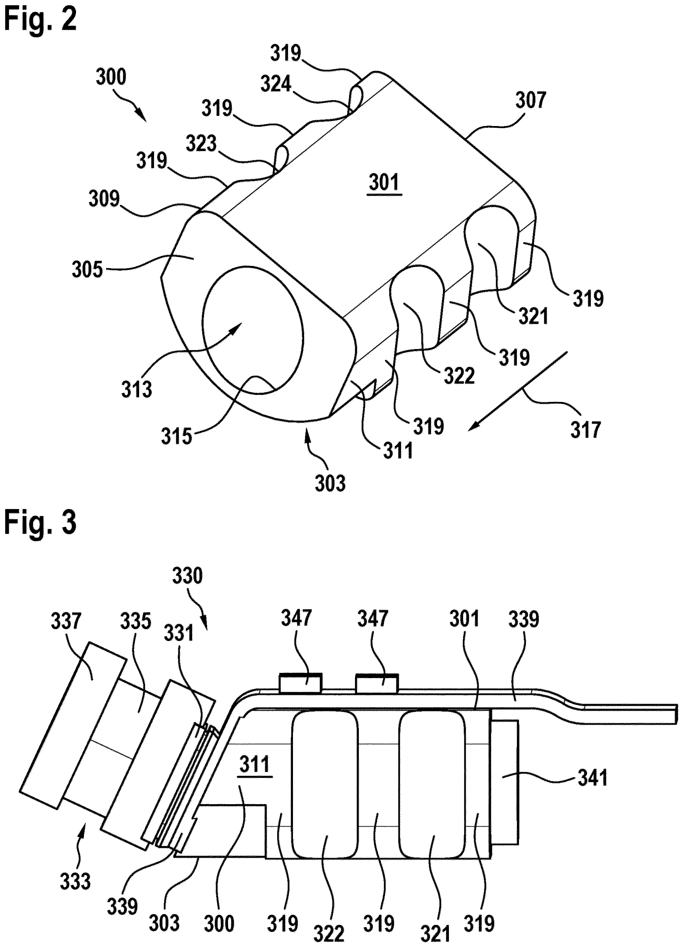

In einem montierten Zustand ist an der Sensorhalterung 300 eine Sensorbaugruppe 330 angeordnet (Figur 3). Die Sensorbaugruppe 330 weist einen elektronischen Bildsensor 331, ein Objektiv 333 mit einer Objektivfassung 335 und eine Platine 339 auf. An der Objektivfassung 335 ist distalseitig ein Deckglas 337 angeordnet. Im montierten Zustand ist in dem Hohlraum 313 der Sensorhalterung 300 ein Kühlkörper 341 ausgebildet als Kupferdraht eingesetzt, welcher distalseitig an die Rückseite des Bildsensors 331 anstößt.

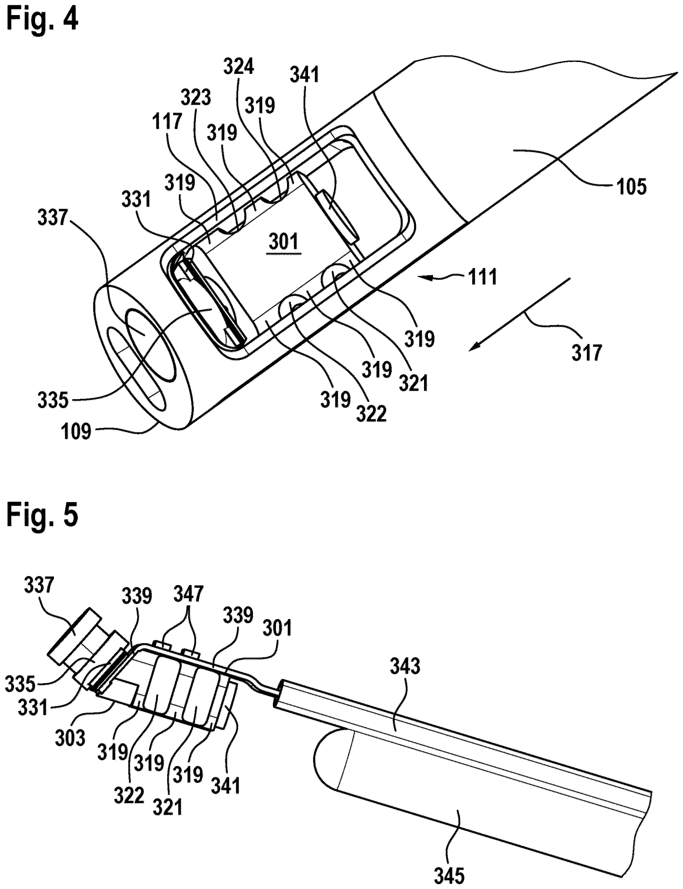

In einem in Figur 4 gezeigten Zustand ist die vormontierte Sensorgruppe 330 an der Sensorhalterung 300 in den Hohlraum des Schaftes 105 in dem distalen Endabschnitt 111 durch die Montageöffnung 117 eingesetzt. Aus Darstellungsgründen ist die Oberseite 301 der Sensorhalterung 300 sichtbar, jedoch nicht die darauf montierte Platine 339.

Die Platine 339 weist mehrere elektronische Bauteile 374 an ihrer Oberseite auf und ist proximalseitig in einem Verbindungsrohr 343 elektrisch mit dem Handstück 103 des Videoendoskops 101 verbunden. Unterhalb des Verbindungsrohrs 343 ist ein Wärmeleitrohr 345 angeordnet. Zwischen dem distalen Ende des Wärmeleitrohrs 345 und dem proximalen Ende des Kühlkörpers 341 ist eine in Figur 5 nicht gezeigte Wärmeleitpaste angeordnet.

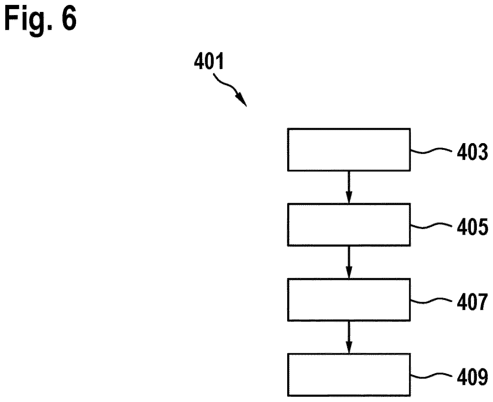

In einem Verfahren 400 zum Vormontieren der Sensorbaugruppe 330 werden mit der Sensorhalterung 300 folgende Arbeitsschritte durchgeführt (Fig. 6):
Außerhalb des Videoendoskopes 101 wird das Objektiv 333 wird an der distalen Seite des Bildsensors 331 durch Verkleben verbunden (Schritt 401). Anschließend wird der mit dem Objektiv 333 verbundene Bildsensor 331 mit seiner proximalen Seite durch Verkleben an der distalen Seite 305 der Sensorhalterung 300 verbunden (Schritt 403). Anschließend wird ein Dorn als Ausrichtwerkzeug von der proximalen Seite 307 durch den Hohlraum 313 der Sensorhalterung 300 eingeführt und der Bildsensor 331 von innen mittels des Dorns ausgerichtet (Schritt 405), bevor die Klebeverbindung ausgehärtet ist. Anschließend werden der derart an der Sensorhalterung 300 vormontierte Bildsensor 331 und das Objektiv 333 werksseitig auf ihre Funktionsfähigkeit geprüft. Nach positiver Prüfung wird der Kühlkörpers 341 durch die proximale Öffnung des Hohlraums 313 in den Hohlraum 313 eingesetzt, sodass distalseitig der Kühlkörper 341 eine Rückseite des Bildsensors 331 kontaktiert. Anschließend wird die Sensorhalterung 300 mit der vormontierten Sensorbaugruppe 330 in den Hohlraum des Schaftes 305 durch die Montageöffnung 117 in eine vorgegebene eingesetzt (Schritt 407). Beim Einsetzen wird die vormontierte Sensorbaugruppe 330 an der Sensorhalterung 300 in distaler Richtung 317 soweit verschoben worden, bis das Deckglas 337 in eine vorgesehene Aufnahme am distalen Ende 109 des Schaftes 105 eingesetzt ist und dort verklebt wird (Figur 4). Mittels einer Kanüle wird nachfolgend von oben in die erste Aussparung 321, die zweite Aussparung 322, die dritte Aussparung 323 und die vierte Aussparung 324 ein UV-Kleber eingebracht, sodass ein Verkleben im Bereich der Aussparungen 321 bis 324 und der Außenseiten der Rippen 319 mit der Innenoberfläche des Schaftes 105 erfolgt (Schritt 409). Anschließend wird ein nicht gezeigter Deckel an der Montageöffnung 117 verschweißt, sodass der Schaft 105 in seinem distalen Endabschnitt 111 fluiddicht verschlossen ist.

Im anschließend Betrieb des Videoendoskops 101 wird die vom Bildsensor 331 abgegebene Betriebswärme durch direkten Kontakt mit der distalen Seite des Kühlkörpers 341 zur proximalen Seite des Kühlkörpers 341 angeordnet innerhalb der Sensorhalterung 300 übertragen. Dort steht der Kühlkörper 341 mit der Wärmeleitpaste in Kontakt, welche zwischen dem Kühlkörper 341 und dem Wärmeleitrohr 345 angeordnet ist, sodass die Wärme über die Wärmeleitpaste weiter auf das Wärmeleitrohr 345 übertragen und in proximaler Richtung abgeführt wird.

Somit wird eine Sensorhalterung 300 bereitgestellt, welche aufgrund ihrer Ausbildung mit dem Hohlraum 313 eine optimale Vormontage der Sensorgruppe 330 und ein Ausrichten und eine Funktionsprüfung des Bildsensors 331 außerhalb des Schaftes 105 ermöglicht, bevor für eine Betriebsfunktion der Kühlkörper 341 in dem Hohlraum 313 eingesetzt und die vormontierte Sensorbaugruppe 330 mit der Sensorhalterung 300 in den distalen Endabschnitt 111 des Schaftes 105 eingesetzt und mittels der Aussparungen 321 bis 324 und der Rippen 319 optimal an die Innenoberfläche des Schaftes 105 verklebt wird. Dadurch wird eine multifunktionale Sensorhalterung 300 bereitgestellt, welche sowohl bei der Vormontage als auch im Betrieb des Videoendoskopes 101 vorteilhaft nutzbar ist.

### Bezugszeichenliste

- 101: Videoendoskop
- 103: Handstück
- 105: Schaft
- 107: proximales Ende des Schaftes
- 109: distales Ende des Schaftes
- 111: distaler Endabschnitt
- 113: Kabel
- 115: Bedienelement
- 117: Montageöffnung
- 201: Anzeigesystem
- 203: Monitor
- 215: Bedienelement
- 300: Sensorhalterung
- 301: Oberseite
- 303: Unterseite
- 305: distale Seite
- 307: proximale Seite
- 309: rechte Seite
- 311: linke Seite
- 313: Hohlraum
- 315: distale Öffnung
- 317: distale Richtung
- 319: Rippe
- 321: erste Aussparung
- 322: zweite Aussparung
- 323: dritte Aussparung
- 324: vierte Aussparung
- 330: Sensorbaugruppe
- 331: Bildsensor
- 333: Objektiv
- 335: Objektivfassung
- 337: Deckglas
- 339: Platine
- 341: Kühlkörper
- 343: Verbindungsrohr
- 345: Wärmeleitrohr
- 347: elektronisches Bauteil
- 400: Verfahren zum Vormontieren einer Sensorbaugruppe
- 401: Verbinden eines Objektivs an dem Bildsensor
- 403: Verbinden des Bildsensors mit einer distalen Seite der Sensorhalterung
- 405: Optional Ausrichten des Bildsensors
- 407: Einsetzen der Sensorhalterung in den Schaft
- 409: Verkleben der Sensorhalterung mit dem Schaft

## Patentansprüche

1. Trägervorrichtung (300) zum Vormontieren und Halten einer Sensorbaugruppe (330) und zum Einsetzen in einen Schaft (105) einer medizinischen Bildgebungsvorrichtung (101), wobei die Trägervorrichtung (300) als eine jeweilige Seite eine distale Seite (305), eine proximale Seite (307), eine Oberseite (301), eine Unterseite (303) und zwei sich gegenüberliegende Seiten (309, 311) aufweist, wobei die Sensorbaugruppe (330) der Trägervorrichtung (300) zuordenbar ist und mindestens einen Sensor (331) aufweist und der Sensor (331) an der distalen Seite (305) der Trägervorrichtung (300) montierbar ist, **dadurch gekennzeichnet, dass** die Trägervorrichtung (300) von der proximalen Seite (307) bis zur distalen Seite (305) einen durchgehenden Hohlraum (313) aufweist, sodass bei einer Vormontage des Sensors (331) an der distalen Seite (305) der Trägervorrichtung (300) der Sensor (331) mittels eines in den Hohlraum (313) einführbaren Ausrichtwerkzeuges von innen in eine vorgegebene Position ausrichtbar ist.

2. Trägervorrichtung (300) nach Anspruch 1, **dadurch gekennzeichnet, dass** die Sensorbaugruppe (330) und/oder die Trägervorrichtung (300) den Sensor (331), eine Objektivfassung (335), ein Objektiv (333) und/oder einen Träger (339) für elektronische Bauteile aufweist oder aufweisen.

3. Trägervorrichtung (300) nach Anspruch 2, **dadurch gekennzeichnet, dass** der Träger (339) für elektronische Bauteile an der Oberseite (301) oder der Unterseite (303) der Trägervorrichtung (300) angeordnet ist.

4. Trägervorrichtung (300) nach einem der vorherigen Ansprüche, **dadurch gekennzeichnet, dass** die Trägervorrichtung (300) mindestens ein Wärmeübertragungselement (341) aufweist oder als ein Wärmeübertragungselement (341) ausgebildet ist.

5. Trägervorrichtung (300) nach Anspruch 4, **dadurch gekennzeichnet, dass** das mindestens eine Wärmeübertragungselement (341) zumindest teilweise in dem Hohlraum (313) angeordnet ist, sodass eine Betriebswärme des Sensors (331) in einer proximalen Richtung abführbar ist.

6. Trägervorrichtung (300) nach einem der vorherigen Ansprüche, **dadurch gekennzeichnet, dass** die Trägervorrichtung (300) in mindestens einer Seite der jeweiligen Seiten (301, 303, 305, 307, 309, 311) mindestens eine Aussparung (321, 322, 323, 324) zum Aufnehmen eines Klebemittels aufweist, sodass die Sensorbaugruppe (330) an die Trägervorrichtung (300) außerhalb des Schaftes (105) vormontierbar und anschließend in den Hohlraum (313) des Schaftes (105) einsetzbar und verklebbar ist.

7. Trägervorrichtung (300) nach Anspruch 6, **dadurch gekennzeichnet, dass** die Trägervorrichtung (300) zwei Aussparungen, drei Aussparungen, vier Aussparungen und optional weitere Aussparungen (321, 322, 323, 324) aufweist.

8. Trägervorrichtung (300) nach Anspruch 7, **dadurch gekennzeichnet, dass** die Aussparungen (321, 322, 323, 324) an den sich gegenüberliegenden Seiten (309, 311) angeordnet sind.

9. Trägervorrichtung (300) nach Anspruch 7 oder 8, **dadurch gekennzeichnet, dass** zwischen zwei Aussparungen (321, 322, 323, 324) eine Rippe (319) angeordnet ist, wobei mittels einer Außenoberfläche der Rippe (319) eine Innenoberfläche des Schaftes (105) kontaktierbar ist.

10. Medizinische Bildgebungsvorrichtung (101), insbesondere Videoendoskop, mit einem Handstück (103) und einem langgestreckten Schaft (105), wobei die medizinische Bildgebungsvorrichtung (101) eine Lichtquelle zum Beleuchten eines Betrachtungsbereiches und der langgestreckte Schaft (105) einen Hohlraum, einen elektrischen Leiter und/oder einem Arbeitskanal aufweist, **dadurch gekennzeichnet, dass** die medizinische Bildgebungsvorrichtung (101) eine Trägervorrichtung (300) nach einem der Ansprüche 1 bis 9 aufweist und der Sensor (331) als ein elektronischer Bildsensor zum Aufnehmen des Betrachtungsbereiches ausgebildet ist.

11. Medizinische Bildgebungsvorrichtung (101) nach Anspruch 10, **dadurch gekennzeichnet, dass** der Schaft (105) eine verschließbare Öffnung (117) zum Einsetzen der Trägervorrichtung (300) und/oder der Sensorbaugruppe (330) aufweist.

12. Medizinische Bildgebungsvorrichtung (101) nach Anspruch 10 oder 11, **dadurch gekennzeichnet, dass** in dem Schaft (105) mindestens ein Wärmeleitkörper (345) ausgerichtet in distaler Richtung (317) derart angeordnet ist, dass das mindestens eine Wärmeübertragungselement (341) der Trägervorrichtung (300) wärmeübertragend mit dem Wärmeleitkörper (345) gekoppelt ist.

13. Verfahren zum Vormontieren einer Sensorbaugruppe an einer Trägervorrichtung und zum Einsetzen in einen Schaft einer medizinischen Bildgebungsvorrichtung, wobei die Sensorbaugruppe mindestens einen Sensor aufweist, die Trägervorrichtung als eine jeweilige Seite eine distale Seite, eine proximale Seite, eine Oberseite, eine Unterseite und zwei sich gegenüberliegende Seiten aufweist und die Trägervorrichtung von der proximalen Seite bis zur distalen Seite einen durchgehenden Hohlraum aufweist, mit folgenden Schritten:
- Verbinden eines Objektivs an dem Sensor, sodass das Objektiv distalseitig von dem Sensor angeordnet ist,
- Verbinden des Sensors mit einer distalen Seite der Trägervorrichtung, und
- Optional Einführen eines Ausrichtwerkzeuges in den Hohlraum der Trägervorrichtung in einer distalen Richtung und Ausrichten des Sensors von innen an der distalen Seite der Trägervorrichtung mittels eines Ausrichtwerkzeuges, und
- Einsetzen der Trägervorrichtung in den Schaft der medizinischen Bildgebungsvorrichtung.

14. Verfahren nach Anspruch 13, **dadurch gekennzeichnet, dass** vor dem Einsetzen in den Schaft ein Verbinden eines Trägers für elektronische Bauteile mit einer Oberseite oder Unterseite der Trägervorrichtung und/oder ein Einführen eines Wärmeübertragungselement zumindest teilweise in den Hohlraum der Trägervorrichtung durchgeführt wird oder werden.

15. Verfahren nach Anspruch 13 oder 14, **dadurch gekennzeichnet, dass** vor oder nach dem Einsetzen ein Einbringen eines Klebemittels in mindestens eine Aussparung in einer Seite der Trägervorrichtung durchgeführt wird, sodass die Trägervorrichtung mit einer Innenseite des Schaftes verklebt wird.
